# EUROPÄISCHE PATENTSCHRIFT

(11) **EP 2 773 295 B1**
(45) Veröffentlichungstag und Bekanntmachung des Hinweises auf die Patenterteilung: **14.02.2018**
(21) Anmeldenummer: 12788117.5
(22) Anmeldetag: 30.10.2012
(51) Int. Cl.: A61F 2/80, A61F 2/78, B29C 70/06, A61F 2/50, A61F 2/60, A61F 2/76

(54) **PROTHESENSCHAFT**
PROSTHESIS SHAFT
EMBOÎTURE DE PROTHÈSE

(30) Priorität: 03.11.2011 DE 102011117801
(43) Veröffentlichungstag der Anmeldung: 10.09.2014
(73) Patentinhaber: Otto Bock HealthCare GmbH, 37115 Duderstadt (DE)
(72) Erfinder: GOTTLIEB, Harald, 37345 Jützenbach (DE); VOLKMAR, Jens, 37115 Duderstadt OT Gerblingerode (DE); LEINIGER, Andreas, 37327 Leinefelde OT Birkungen (DE); VOLKMAR, Marco, 37115 Duderstadt (DE)
(74) Vertreter: Friedrich, Andreas
(86) Internationale Anmeldenummer: PCT/EP2012/004536
(87) Internationale Veröffentlichungsnummer: WO 2013/064240

(56) Entgegenhaltungen:
- EP-A1- 1 854 621
- DE-A1-102006 046 927
- US-A- 5 653 766
- US-A1- 2004 158 332
- US-A1- 2010 191 348

## Beschreibung

Die Erfindung betrifft einen Prothesenschaft für eine Prothese, der Anschlussmittel für eine distale Protheseneinrichtung und einen Außenschaft umfasst, der überwiegend aus einem starren Material gebildet ist und eine proximale Öffnung zum Aufnehmen eines Amputationsstumpfes umfasst, die von einem Außenschaftrand umgeben ist. Derartige Prothesenschäfte sind beispielsweise aus der DE 10 2006 046 927 A1 sowie der US 2010/0191348 A1 bekannt.

Derartige Prothesenschäfte sind aus dem Stand der Technik seit langem bekannt und werden insbesondere bei Beinprothesen, beispielsweise bei Amputationen oberhalb des Knies des Patienten, verwendet.

Bei bekannten Prothesenschäften besteht der Außenschaft beispielsweise vollständig aus einem faserverstärkten Kunststoff, beispielsweise einem kohlefaser- oder glasfaserverstärkten Kunststoff. Diese Materialien sind leicht zu bearbeiten, stabil und weisen dennoch ein sehr geringes Eigengewicht auf. Auf diese Weise ist eine besonders leichte Prothese bzw. ein leichter Prothesenschaft erreichbar, der dennoch über gute Stabilitätseigenschaften verfügt. Insbesondere durch die hohe Eigenstabilität des Außenschaftes wird dem Träger der Prothese ein Gefühl der Sicherheit vermittelt. Durch die hohe Stabilität und Festigkeit des Außenschaftes ist zudem eine sichere und stabile Anbindung distaler Protheseneinrichtungen, wie beispielsweise eines Prothesenfußes oder eines Kniegelenks mit anhängendem Prothesenunterschenkel und -fuß, möglich.

Nachteilig ist jedoch, dass ein Amputationsstumpf, der sich in einem derartigen Prothesenschaft befindet, von Wahrnehmungen der Umwelt weitestgehend oder sogar völlig abgeschottet wird. Setzt sich der Träger einer Prothese, die mit einem gattungsgemäßen Prothesenschaft ausgerüstet ist, beispielsweise hin und stützt die Arme auf die Oberschenkel, hat er aufgrund des starren Außenschaftes des Prothesenschaftes keine Möglichkeit, von seinem Amputationsstumpf eine entsprechende Rückmeldung zu erhalten. Daher kommt es zu einem unnatürlichen und künstlichen Gefühl, das den Tragekomfort einer mit einem derartigen Prothesenschaft ausgestatteten Prothese deutlich mindert.

Aus der EP 0 760 640 B1 ist daher ein Prothesenschaft bekannt, bei dem der Außenschaft lediglich in Form einer Aufnahmeschale gebildet ist, in der das distale Ende des Amputationsstumpfes eingesetzt wird. Von dieser Aufnahmeschale ausgehend erstrecken sich drei Schaftfinger zum proximalen Ende des Prothesenschaftes. Diese Schaftfinger sind aus einem starren und haltbaren Material gefertigt und weisen an der Innenseite, also der dem Amputationsstumpf zugewandten Seite, einen Klettverschluss auf. Über den Amputationsstumpf selbst wird ein Innenschaft gezogen, der an entsprechenden Stellen ebenfalls über Klettverschlusselemente verfügt. Durch ein Zusammenwirken der Klettverschlusselemente am Innenschaft und an der Innenseite des Außenschaftes wird der Außenschaft am Innenschaft festgelegt. Nachteilig ist jedoch zum einen, dass es aufgrund der Verwendung von Klettverschlüssen zu Relativbewegungen zwischen dem Innenschaft und dem Außenschaft kommen kann, insbesondere in der Schwungphase einer Gehbewegung. Nachteilig ist zum anderen, dass insbesondere im proximalen Bereich des Prothesenschaftes relativ wenig Halt vorhanden ist, da der starre Außenschaft sich nicht bis in diesen Bereich erstreckt. Für den Patienten ergibt sich somit gegebenenfalls ein Gefühl unzureichender Kontrolle über die Prothese, was zu einer erhöhten Unsicherheit und damit zu einer Verminderung des Tragekomforts führt.

Aus der US 5,246,464 ist ein Prothesenschaft bekannt, der über einen starren Außenschaft verfügt, der an einigen Stellen durchbrochen ist. Dennoch verfügt der Außenschaft über einen Außenschaftrand, der den gesamten Umfang des Amputationsstumpfes umläuft, und das proximale Ende bzw. den proximalen Rand des Außenschaftes definiert. Durch die vorgesehenen Durchbrechungen im starren und festen Außenschaft kann ein sich in dem Prothesenschaft befindender Amputationsstumpf die Umwelt zumindest in begrenztem Maße wahrnehmen. Insbesondere am Amputationsstumpf anliegende Gegenstände oder andere Körperteile des Patienten, wie beispielsweise aufgestützte Ellbogen, können innerhalb dieser Fenster, die in dem Außenschaft vorgesehen sind, wahrgenommen werden. Da ein derartiger Außenschaft jedoch über einen über den gesamten Umfang umlaufenden Außenschaftrand verfügt, kann es hier noch immer zu Druckstellen und Einschneidungen kommen, die zu schmerzhaften Wunden oder Blessuren führen können.

Die DE 10 2006 046 927 A1, die US 2004/0158332 A1, die US 2010/0191348 A1 und die US 5,653,766 offenbaren jeweils einen Prothesenschaft, der über starre Schalenelemente verfügt, die über Gurte und Gurtelemente aneinander befestigt werden können, sodass auf diese Weise der Prothesenschaft am Amputationsstumpf des Trägers befestigt werden kann. Dabei ist jeweils ein Innenschaft vorgesehen, der aus einem elastischen Material besteht, um den Tragekomfort zu erhöhen.

Die EP 1 854 621 A1 offenbart ein Verfahren zum Gießen eines Prothesenschaftes, bei dem unterschiedliche weich-elastische Polyurethanlagen verwendet werden. Aus dem Stand der Technik sind daher auch Prothesenschäfte bekannt, die über einen starren Außenschaft verfügen, wobei jedoch der Außenschaftrand, der die proximale Öffnung des Außenschaftes begrenzt, zumindest teilweise durch Gurte gebildet wird. Da diese zumindest in diesen Bereichen des Außenschaftrandes für die Stabilität des Außenschaftes selbst und für die sichere Anordnung des Prothesenschaftes am Amputationsstumpf verantwortlich sind, müssen diese Gurte relativ stramm und fest angezogen werden. Dadurch kommt es zu einem erhöhten Druck auf den Amputationsstumpf im Vergleich zu einem beispielsweise vollständig aus einem starren Material bestehenden Außenschaft. Dieser erhöhte Druck auf den Amputationsstumpf im Bereich der Gurte kann für den Träger sehr unangenehm und gegebenenfalls sogar schmerzhaft werden und zu Druckstellen und Blessuren führen.

Der Erfindung liegt daher die Aufgabe zugrunde, einen Prothesenschaft vorzuschlagen, mit dem eine verbesserte Wahrnehmung der Umwelt durch den Amputationsstumpf ermöglicht wird und der gleichzeitig einen sicheren Halt am Amputationsstumpf gewährleistet und auch bei unterschiedlichen Bewegungen nicht zu Druckstellen führt und die Steuerfähigkeit nicht beeinträchtigt.

Die Erfindung löst die gestellte Aufgabe durch einen Prothesenschaft der eingangs erwähnten Art, bei dem der Außenschaftrand in einem ventralen Bereich aus einem flexiblen und unelastischen Material gebildet ist, das so ausgebildet ist, dass es unter Einwirkung der herkömmlicherweise beim Tragen des Prothesenschaftes auftretenden Belastungen zu flexiblen Verformungen, nicht jedoch zu einer Aufweitung der proximalen Öffnung kommt, wobei das flexible und unelastische Material an eine erwartete Körperform eines Trägers des Prothesenschaftes anpassbar ist, wobei diese Form nach Ausformen des flexiblen und unelastischen Materials eine Ruheposition des flexiblen und unelastischen Materials bildet, in die es zurückkehrt, sofern es nicht anderen Kräften unterworfen ist.

Derartige Materialien, insbesondere Dyneema®-verstärkter Kunststoff sind in einem ersten, nicht ausgehärteten Zustand, frei formbar. Sie können insbesondere an die Körperform des späteren Trägers des Prothesenschaftes oder der Prothese angepasst werden. Dabei bedeutet im Kontext der vorliegenden Erfindung eine Anpassung des Materials an die Körperform des Trägers des Prothesenschaftes nicht zwangsläufig, dass eine individuelle Anpassung an jeden einzelnen Träger der Prothese bzw. des Prothesenschaftes vorgenommen werden muss. Vielmehr umfasst die gewählte Formulierung auch eine nur standardmäßige Anpassung des flexiblen und unelastischen Materials an eine erwartete Körperform des Trägers. Diese kann beispielsweise eine Austulpung des Außenschaftrandes umfassen, um so potentielle Druckstellen abzumindern und Blessuren vorzubeugen. Eine derartige Anpassung ist durch eine zumindest teilweise Ausbildung des Schaftrandes durch Gurte nicht zu erreichen.

Nach dem Ausformen des flexiblen und unelastischen Materiales entsprechend der erwarteten Körperform des Trägers des Prothesenschaftes bildet diese Form die Ruheposition des flexiblen und unelastischen Materials. Dies bedeutet, dass das Material, sofern es nicht anderen Kräften unterworfen ist, in diese Position zurückkehrt. Aufgrund der speziellen Eigenschaften des Materials, insbesondere des Dyneema-faserverstärkten Kunststoffes, hat sich das Material jedoch auch im ausgehärteten Zustand eine Restflexibilität bewahrt, so dass es unter Einwirkung der herkömmlicherweise beim Tragen eines Prothesenschaftes der vorliegenden Art auftretenden Belastungen zu flexiblen Verformungen kommt. Gleichzeitig ist das Material jedoch unelastisch, so dass es beispielsweise nicht zu einer Aufweitung der proximalen Öffnung des Prothesenschaftes kommen kann, wenn eine besondere Belastung des flexiblen und unelastischen Materials vorliegt. Dadurch wird ein sicherer Halt des Prothesenschaftes am Amputationsstumpf des Trägers des Prothesenschaftes gewährleistet.

Wird ein erfindungsgemäßer Prothesenschaft beispielsweise für die Prothese eines Patienten verwendet, dessen Bein oberhalb des Knies amputiert werden musste, kommt es insbesondere im Bereich des Außenschaftrandes zu einer erhöhten Belastung. Bei Prothesenschäften aus dem Stand der Technik kommt es dadurch in diesem Bereich am Amputationsstumpf zu Druckstellen und gegebenenfalls schmerzhaften Wunden. Durch die erfindungsgemäße Ausgestaltung hingegen ist der Außenschaft im Bereich des Außenschaftrandes durch das flexible und unelastische Material jedoch auch flexibel genug, um aufgrund der in diesem Fall auftretenden Belastungen seine Form zu ändern. Die Gefahr von Druckstellen oder durch Scheuern hervorgerufenen Wunden wird auf diese Weise deutlich verringert.

Gleichzeitig ist es für den Träger des Prothesenschaftes bzw. einer damit ausgerüsteten Prothese aufgrund der Flexibilität des in diesem Bereich des Außenschaftes verwendeten Materials möglich, Einflüsse der Umgebung, wie beispielsweise auf den Prothesenschaft abgelegte Gegenstände oder Körperteile, wahrzunehmen und somit ein natürlicheres Geführ für die Prothese zu entwickeln. Dadurch steigt der Tragekomfort der Prothese und des Prothesenschaftes, so dass ein natürlicherer Umgang mit der Prothese möglich wird.

Als bevorzugtes flexibles und unelastisches Material wird ein mit Dyneemafasern verstärkter Kunststoff verwendet. Bei Dyneema-Fasern handelt es sich um Polyethylenfasern, die hoch überstreckt sind. Vergleichbare Fasern werden beispielsweise unter der Markenbezeichnung "Spektra" am Markt angeboten. Derartige Fasern und mit ihnen verstärkter Kunststoff sind besonders einfach zu verarbeiten und weisen alle benötigten Eigenschaften auf. Alternativ können auch Polyamidfasern, beispielsweise Kevlar, verwendet werden. Dabei ist es nicht nötig, dass die Fasern über die gesamte Fläche mit einem Kunststoff beschichtet oder mit diesem getränkt oder in diesen eingebettet sind. In den Randbereichen der durch die Fasern gebildeten Flächen, also der Flächen, die durch das flexible und unelastische Material gebildet werden, ist die Verwendung eines derartigen Kunststoffs jedoch vorteilhaft, da so eine einfache Anbindung an das starre Material des Außenschaftes ermöglicht wird.

In einer bevorzugten Ausführungsform ist das starre Material des Außenschaftes ebenfalls ein faserverstärkter Kunststoff. Dabei handelt es sich vorteilhafterweise um einen glasfaser- oder kohlefaserverstärkten Kunststoff. Diese Materialien weisen die bereits genannten Vorteile des geringen Eigengewichtes, der hohen Stabilität und der leichten Verarbeitbarkeit auf. Über die hohe Eigenstabilität des Außenschaftes bleibt die Steuerfähigkeit der Prothese erhalten, wobei insbesondere bei einem Oberschenkelschaft eine rigide Anlage für die Steuerung vorteilhaft ist.

In einer besonders bevorzugten Ausführungsform kann für das starre Material und das flexible und unelastische Material der gleiche Kunststoff verwendet werden. Dadurch kann eine dafür vorgesehene Form in einem Arbeitsschritt in den jeweiligen Bereichen mit den jeweiligen faserverstärkten Kunststoff ausgelegt werden. Zudem können die beiden faserverstärkten Kunststoffe gleichzeitig aushärten und so eine besonders feste, insbesondere stoffschlüssige Verbindung eingehen. Dadurch kommt es zu einer erhöhten Stabilität insbesondere im Kontaktbereich der beiden faserverstärkten Kunststoffe am Prothesenschaft. Dadurch wird die Lebensdauer des Prothesenschaftes deutlich verlängert. Zudem entsteht zwischen den beiden aneinander angrenzenden Materialien keine Grenzschicht oder gar ein Absatz, der den Tragekomfort eines derartigen Prothesenschaftes deutlich verringert würde.

Vorzugsweise ist der Außenschaftrand in einem dorsalen Bereich aus dem flexiblen und unelastischen Material gebildet. Insbesondere bei Patienten, deren Bein oberhalb des Knies amputiert werden musste, ist der dorsale, also hintere Bereich des Außenschaftrandes der Bereich, der beim Hinsetzen am meisten belastet wird, wenn dabei die Prothese mit dem beschriebenen Prothesenschaft getragen wird. Der Patient setzt sich praktisch auf den Außenschaftrand. Ist dieser starr, stabil und hart ausgebildet, kommt es auch hier zu einem unangenehmen Druck, der zu Druckstellen oder durch Scheuern sogar zu Wunden führen kann. Dadurch, dass auch im dorsalen Bereich ein flexibles und unelastisches Material verwendet wird, kann diesen Beschwerden vorgebeugt werden. Beim Hinsetzen auf den Außenschaftrand verformt sich das flexible und unelastische Material aufgrund seiner Flexibilität, so dass es die für die herrschenden Belastungen optimale Form einnehmen kann, so dass Druckstellen sicher verhindert werden.

Als besonders vorteilhaft hat sich dabei herausgestellt, wenn das flexible und unelastische Material im dorsalen Bereich des Außenschaftes das gleiche Material ist, wie das flexible und unelastische Material im ventralen Bereich des Außenschaftes. Handelt es sich bei beiden Materialien zudem um einen faserverstärkten, insbesondere Dyneema-faserverstärkten Kunststoff, kann für diesen faserverstärkten Kunststoff beispielsweise wieder der gleiche Kunststoff verwendet werden, wie für den verbleibenden Rest des Außenschaftes, der aus dem starren Material gebildet ist. Auch hier kommt es somit zu den gleichen Vorteilen, die bereits beschrieben wurden, nämlich einer besonders starken, stoffschlüssigen Verbindung und zu einer besonders glatten Innenfläche des Außenschaftes, da Stufen und Absätze zwischen zwei benachbarten Materialien sicher vermieden werden.

Vorteilhafterweise ist der Außenschaft an zumindest einer Stelle durchbrochen. Derartige Durchbrüche, oder Fenster, reduzieren zum einen das Gewicht des Außenschaftes und geben zudem dem Träger des Prothesenschaftes und der Prothese die Möglichkeit, in diesem Bereich Einflüsse der Außenwelt wahrzunehmen. Dabei ist es denkbar, dass die Durchbrüche oder Fenster vollständig mit dem flexiblen und unelastischen Material ausgefüllt sind. Alternativ dazu ist es auch möglich, in diesen Durchbrüchen kein Material vorzusehen, da der Prothesenschaft der Prothese einen über weite Bereiche starren Außenschaft verfügt, ist es vorteilhaft, wenn zwischen dem starren Außenschaft und dem Amputationsstumpf eine polsternde Schicht vorgesehen wird. Diese kann beispielsweise in Form eines Liner oder eines Sleeve über den Amputationsstumpf gezogen werden. Als besonders vorteilhaft hat sich jedoch herausgestellt, wenn innerhalb des Außenschaftes ein Innenschaft aus einem flexiblen Material angeordnet ist. Dies kann beispielsweise ein Silikonschaft sein, der vorteilhafterweise im proximalen Bereich eine Antihaftbeschichtung, beispielsweise mittels CVD-Verfahren, aufweist. Dadurch wird der Einstieg des Patienten in den Innenschaft deutlich erleichtert, weil die Haftreibung des Innenschaftes am Amputationsstumpf in diesem Bereich deutlich reduziert wird. Ein solcher Innenschaft kann beispielsweise zwei Lagen HTV Silikon umfassen. Derartige Innenschäfte können mit polsternden Verdickungen oder an besonders zu polsternden Stellen, beispielsweise geklebte Polsterpads, ausgestattet sein. Diese können gegebenenfalls für jeden Patienten individuell hergestellt, angepasst und angeordnet werden. Vorzugsweise ist im Bereich der Fensterung die Elastizität des Silikons minimiert. Dies wird idealerweise durch die Einarbeitung von Gewebe in die Silikonschichten erreicht. Die proximalen Ränder des Innenschaftes bleiben elastisch verformbar.

Ein Innenschaft umfasst vorzugsweise eine an einem distalen Ende angeordnete integrierte Spange, die aus einem stabilen Material, beispielsweise ein faserverstärkten Kunststoff hergestellt sein kann. Diese verhindert ein Zusammenfallen oder Implodieren des Innenschaftes und bietet gleichzeitig beispielsweise Halt und Stabilität für einen Ventilanschluss eines Ventils, durch das, gegebenenfalls mit einer separaten Pumpe ein Unterdruck zwischen dem Innenschaft und dem Amputationsstumpf hergestellt wird, durch den der Prothesenschaft am Amputationsstumpf gehalten wird.

Werden die Fenster bzw. Durchbrüche im Außenschaft des Prothesenschaftes mit dem flexiblen und unelastischen Material ausgefüllt, ist es für den Träger des Prothesenschaftes weiterhin möglich, hier Umwelteinflüsse wahrzunehmen. Gleichzeitig wird jedoch sicher verhindert, dass Teile des Amputationsstumpfes insbesondere beim Auftreten mit einer derartigen Prothese aus diesen Durchbrüchen und Fenstern herausquellen. Auf diese Weise wird auch ein sogenannter Melkeffekt verhindert und der Tragekomfort der Prothese weiter erhöht.

Dazu ist es von Vorteil, wenn der Innenschaft mit dem Außenschaft hubfrei, verbunden ist. Dies erfolgt in einer besonders vorteilhaften Ausgestaltung durch wenigstens eine insbesondere distale Schraubverbindung, mit der der Innenschaft mit dem Außenschaft verbunden ist. Vorzugsweise ist der Innenschaft distal, insbesondere durch eine Verschraubung, mit dem Außenschaft verbunden.

Bei aus dem Stand der Technik bekannten Verbindungssystemen, beispielsweise über Klettverschlüsse, ist es denkbar, dass insbesondere in der Schwungphase einer Beinprothese die auf die Prothese wirkenden Zugkräfte dazu führen, dass es zwischen dem an der distalen Protheseneinrichtung festgelegten Außenschaft und dem am Amputationsstumpf festliegenden Innenschaft zu einer Relativbewegung kommt. Für den Träger der Prothese fühlt sich eine derartige Relativbewegung so an, als wäre die Prothese lose und nicht in vollständig funktionstüchtiger Weise am Amputationsstumpf festgelegt. Eine derartige Relativbewegung zwischen dem Außenschaft und dem Innenschaft hat somit ein erhebliches Unsicherheitsgefühl zur Folge, das durch die insbesondere hubfreie Befestigung des Innenschaftes am Außenschaft sicher verhindert wird.

In einer bevorzugten Ausführungsform ist es möglich, dass flexible und unelastische Material in zwei Teilen auszubilden, die beispielsweise über einen Klettverschluss oder einen an der Außenseite befestigten Gurt miteinander verbindbar sind. Auf diese Weise können die Vorteile des flexiblen und unelastischen Materials, das der Körperform des Trägers angepasst ist, mit den Vorteilen der Verstellbarkeit über ein Gurtsystem oder eine sonstige Verschließeinrichtung kombiniert werden. Auf diese Weise ist es insbesondere möglich, den Prothesenschaft über einen langen Zeitraum bei einem Patienten zu verwenden, auch wenn sich beispielsweise das Volumen des Amputationsstumpfes über die Jahre ändert. Eine individuelle Anpassung der Größe des Prothesenschaftes ist auf diese Weise in einem begrenzten Rahmen möglich.

Die Größe des ventralen Bereichs, in dem der Außenschaft aus dem flexiblen und unelastischen Material gebildet ist, kann dabei sowohl in Umfangsrichtung als auch in einer Ausdehnung von der proximalen Öffnung hin zum distalen Ende des Prothesenschaftes stark variieren. So ist es möglich, in dieser Richtung die Ausdehnung des ventralen Bereiches auf wenige Zentimeter zu beschränken. Alternativ dazu kann auch ein Bereich mit dem flexiblen und unelastischen Material vorgesehen sein, der sich über mehr als die Hälfte der Ausdehnung des Außenschaftes in dieser Richtung erstreckt.

Mit Hilfe einer Zeichnung wird nachfolgend ein Ausführungsbeispiel der vorliegenden Erfindung näher erläutert. Es zeigt
- Figur 1: - einen Prothesenschaft gemäß einem ersten Ausführungsbeispiel der vorliegenden Erfindung,
- Figur 2: - einen Prothesenschaft gemäß einem Ausführungsbeispiel der vorliegenden Erfindung,
- Figur 3: - einen Prothesenschaft gemäß einem weiteren Ausführungsbeispiel der vorliegenden Erfindung,
- Figur 4: - einen Innenschaft für einen Prothesenschaft gemäß einem Ausführungsbeispiel der vorliegenden Erfindung, und
- Figur 5: - den Innenschaft aus Figur 4 aus einem anderen Blickwinkel.

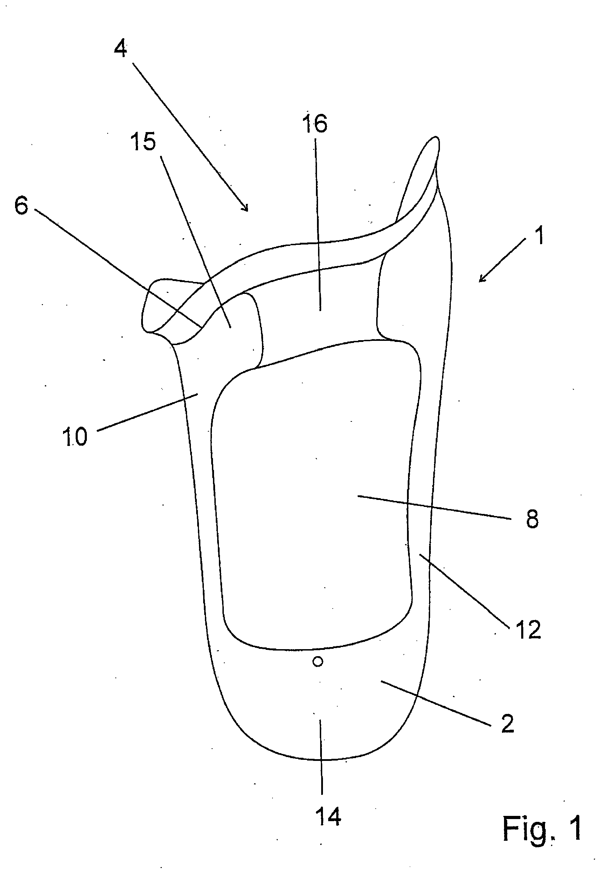

Figur 1 zeigt einen Prothesenschaft 1 gemäß einem Ausführungsbeispiel der vorliegenden Erfindung. Der Prothesenschaft 1 verfügt über einen Außenschaft 2, der eine proximale Öffnung 4 umfasst, die von einem Außenschaftrand 6 umgeben ist. Im Innern des Außenschaftes 2 befindet sich ein Innenschaft 8, der in proximaler Richtung, in Figur 1 also nach oben, über den Außenschaftrand 6 hervorsteht und den Prothesenschaft 1 in proximaler Richtung begrenzt.

Der Außenschaft 2 besteht aus einem starren Material, beispielsweise einem faserverstärktem Kunststoff. Dabei kann es sich beispielsweise um einen Kohlefaserverbundwerkstoff, also einen kohlefaserverstärkten Kunststoff handeln. Durch den in proximaler Richtung über den Außenschaftrand 6 hervorstehenden Innenschaft 8 wird der harte und starre Außenschaftrand 6 für den Träger der Prothese beziehungsweise des Prothesenschaftes 1 gepolstert.

Man erkennt, dass der Außenschaft 2 einen medialen Anteil 10 und einen lateralen Anteil 12 aufweist, die an einem distalen Ende 14 des Außenschaftes 2 miteinander verbunden sind. Im proximalen Bereich sind der mediale Anteil 10 und der laterale Anteil 12 durch ein flexibles und unelastisches Material 16 verbunden. Dieses befindet sich, wie in Figur 1 dargestellt, in einem ventralen Bereich des Außenschaftes 2. Ein proximaler Anteil 15 des medialen Anteils 10 umfasst ca. 1/3 der ventralen Fläche in rigider und unelastische Ausführung, da er aus dem starren Material des Außenschaftes 2 besteht. Durch diese Ausführung beispielsweise durch kohlefaserverstärkten Kunststoff wird die Steuerfähigkeit und die sichere Fixierung am Amputationsstumpf sichergestellt. Zusätzlich wird eine insbesondere dauerhafte Verformung des ventralen flexiblen und unelastischen Materials 16 verhindert.

Alternativ zu der in Figur 1 dargestellten Ausführungsform kann beispielsweise auch der Bereich zwischen dem medialen Anteil 10 und dem lateralen Anteil 12 vollständig mit dem flexiblen und unelastischen Material 16 ausgefüllt sein. Dies hätte den Vorteil, dass ein Herausquillen des Innenschaftes 8 mit dem sich darin befindlichen Amputationsstumpf aus dem zwischen dem medialen Anteil 10 und dem lateralen Anteil 12 gebildeten Fenster verhindert wird. Durch die in Figur 1 gezeigte Ausführungsform wird jedoch die Senomotorik, also die Wahrnehmung der Umwelt, über den Amputationsstumpf deutlich verbessert. Besteht der Innenschaft 8 zudem aus einem Silikon, beispielsweise einem HTV Silikon, wird dessen Atmungsaktivität deutlich verbessert und zudem eine Funktionseinschränkung der Muskulatur des Amputationsstumpfes verringert. Die Elastizität des Silikons wird durch Einarbeitung eines Gewebes eingeschränkt.

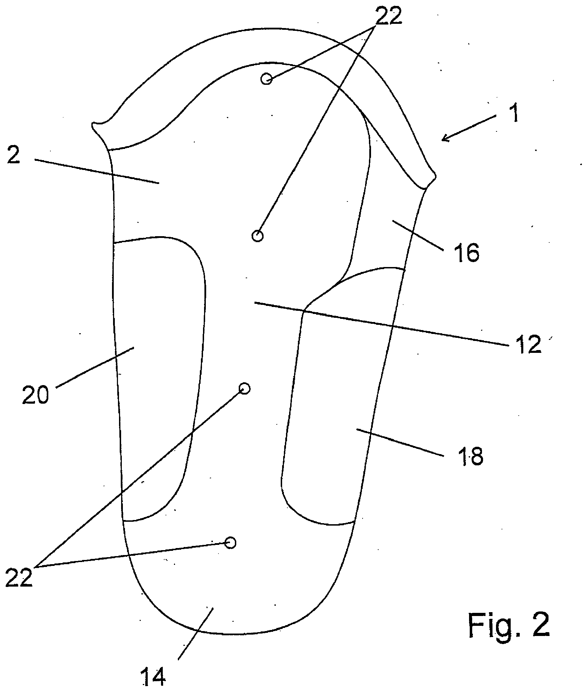

Figur 2 zeigt den Prothesenschaft 1 aus Figur 1 in einer lateralen Ansicht. Man erkennt folglich den lateralen Anteil 12 des Außenschaftes 2 sowie dessen distales Ende 14. Zudem ist zu erkennen, dass in dem Außenschaft 2 nicht nur ein ventrales Fenster 18 vorgesehen ist, das nach oben hin durch das flexible und unelastische Material 16 begrenzt wird, sondern dass auch im dorsalen Bereich, also hinten, ein dorsales Fenster 20 vorgesehen ist. Dies führt insbesondere beim Sitzen mit dem gezeigten Prothesenschaft 1 zu einer deutlich besseren Wahrnehmung der Umgebung über den Prothesenschaft.

Im lateralen Anteil 12 sind zudem mehrere Bohrungen 22 dargestellt, durch die Verbindungselemente, beispielsweise Schraubverbinder, geführt werden können, um den Außenschaft 2 mit dem Innenschaft hubfrei zu verbinden.

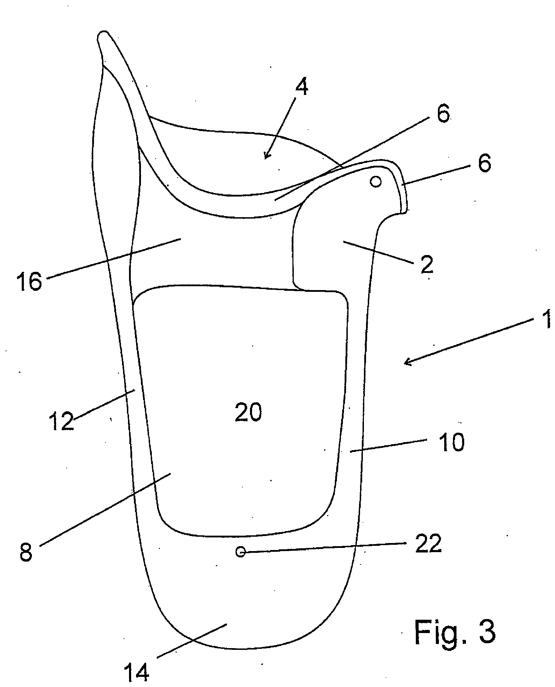

Figur 3 zeigt den Prothesenschaft 1 in einer dorsalen Ansicht. Man erkennt das dorsale Fenster 20 im Außenschaft 2, durch das der Innenschaft 8 erkennbar ist. In Figur 3 ist zudem dargestellt, dass sich auch auf der dorsalen Seite, also hinten, zwischen dem medialen Anteil 10 und dem lateralen Anteil 12 ein Einsatz aus dem flexiblen und unelastischen Material 16 befindet. Dieser sorgt insbesondere beim Sitzen mit einem derartigen Prothesenschaft 1 für ein deutlich angenehmeres Tragegefühl, da er zum einen den auftretenden Belastungen nachgibt und sich den auftretenden Kräften entsprechend verformt und so zu deutlich weniger Druckstellen führt, und zum anderen eine Wahrnehmung der Umgebung erlaubt, die durch ein starres Material nicht möglich wäre. An dem in den Figuren 1 bis 3 gezeigten Prothesenschaft 1 befinden sich am distalen Ende 14 nicht gezeigte Verbindungsmittel, um hier eine distale Protheseneinrichtung am Prothesenschaft 1 zu befestigen.

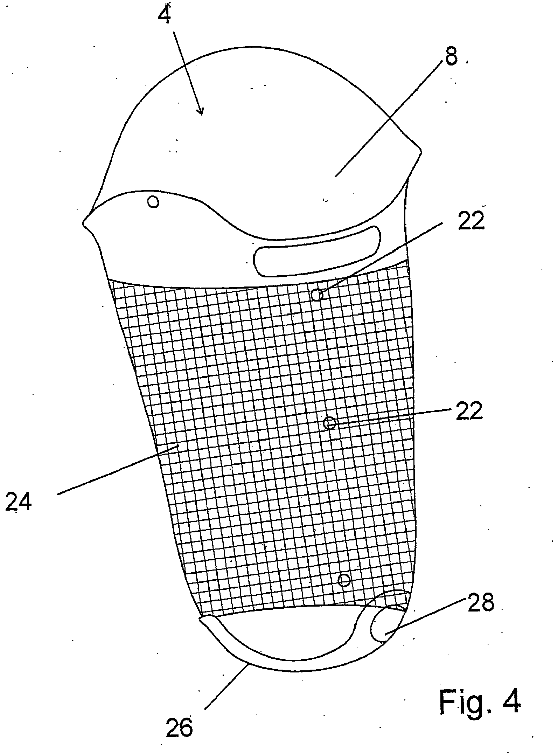

Figur 4 zeigt den Innenschaft 8 für den Prothesenschaft 1 aus den Figuren 1 bis 3. Auch der Innenschaft 8 verfügt über eine proximale Öffnung 4, die zur Aufnahme des Amputationsstumpfes vorgesehen ist. Der Innenschaft 8 besteht aus einem flexiblen und polsternden Material, beispielsweise einer oder mehrerer Schichten Silikon. Über Bohrungen 22, die auf die Position der Bohrungen 22 im Außenschaft 2, wie sie in Figur 2 dargestellt sind, angepasst sind, lässt sich der Außenschaft 6 mit dem Innenschaft 8 hubfrei verbinden. Man erkennt im mittleren Bereich des Innenschaftes 8 eine bistabile Matrix 24, die durch sich kreuzende Linien dargestellt ist. Diese bistabile Matrix 24 unterbindet die Elastizität des Innenschafts 8 in diesem Bereich. Da der Außenschaft 2 in diesem Bereich sein ventrales Fenster 18 bzw. sein dorsales Fenster 20 aufweist, kann es bei einer zu großen Elastizität des Innenschaftes 8 in diesem Bereich zu einem Herausquillen des Innenschaftes 8 mit dem sich darin befindlichen Amputationsstumpf kommen. Durch die bistabile Matrix 24 wird dies sicher verhindert und so zum einen der Halt des Prothesenschaftes 1 am Amputationsstumpf verbessert und zum anderen der Tragekomfort erhöht.

Im distalen Bereich verfügt der in Figur 4 gezeigte Innenschaft 8 über eine distale Spange 26, die aus einem starren festen Material besteht, das beispielsweise der gleiche faserverstärkte Kunststoff sein kann, aus dem auch der Außenschaft 2 besteht. Diese distale Spange 26 verhindert ein Kollabieren oder Zusammenfallen des distalen Endes des Innenschaftes 8 und bietet gleichzeitig Halt für ein Ventil, das in eine dafür vorgesehene Ventilöffnung 28 einsetzbar ist. Dieses kann beispielsweise ein herkömmliches Auslassventil sein, durch das allein durch die beim Gehen mit dem Prothesenschaft 1 auftretende Pumpbewegung eventuell zwischen dem Innenschaft 8 und dem Amputationsstumpf befindliche Luft aus dem Zwischenraum herausgepumpt wird. Dadurch wird ein Unterdruck zwischen dem Innenschaft 8 und dem Amputa-tionsstumpf aufrecht erhalten und so eine feste Verbindung zwischen dem Amputationsstumpf und dem Prothesenschaft 1 erreicht.

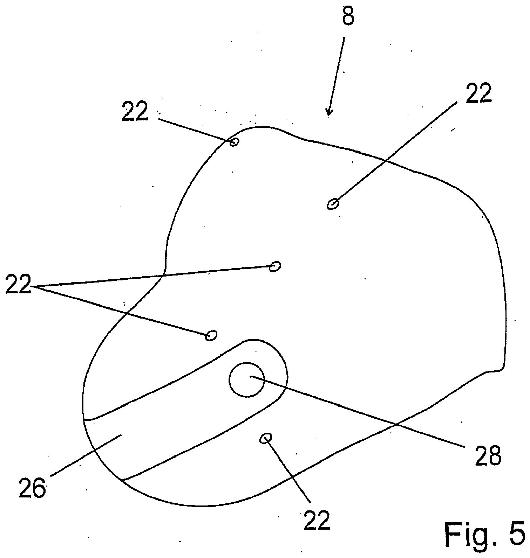

Figur 5 zeigt den Innenschaft 8 aus einer leicht geänderten Perspektive. Man erkennt eine Mehrzahl Bohrungen 22, in die beispielsweise Setzmuttern eingesetzt werden können, die mit in die Bohrungen 22 im Außenschaft 2 eingesetzten Schrauben zusammenwirken und so den Innenschaft 8 mit dem Außenschaft 2 hubfrei verbinden. Im distalen Bereich des Innenschaftes 8 ist wieder die distale Spange 26 mit der darin vorgesehenen Ventilöffnung 28 dargestellt. Auf eine erneute Darstellung der bistabilen Matrix 24 wurde aus Gründen der Übersichtlichkeit verzichtet.

In einer bevorzugten Ausführungsform verfügt der Innenschaft 8 auf seiner Innenseite über eine gleitende Innenbeschichtung, die beispielsweise mittels eines CVD-Verfahrens aufgebracht werden kann. Damit wird eine Reibung und eine damit verbundene Hautreizung zwischen dem proximalen Ende des Prothesenschaftes 1, das durch den Innenschaft 8 gebildet wird, und der Haut des Patienten vermieden. Eine gleitende Beschichtung auf der Außenseite des Innenschaftes 8 ist insbesondere im proximalen Anteil des Innenschaftes 8, der den Außenschaftrand 6 proximal überragt, sinnvoll. Auf diese Weise wird eine Reibung und eine Haftung zwischen dem Innenschaft 8 und der Kleidung des Patienten verhindert. Gleichzeitig wird durch eine gleitende Beschichtung des Innenschaftes 8 an dessen Innenseite der Einstieg des Patienten in den Prothesenschaft 1 deutlich erleichtert.

### Bezugszeichenliste

- 1: Prothesenschaft
- 2: Außenschaft
- 4: Proximale Öffnung
- 6: Außenschaftrand
- 8: Innenschaft
- 10: Medialer Anteil
- 12: Lateraler Anteil
- 14: Distales Ende
- 15: Proximaler Bereich
- 16: Flexibles und unelastisches Material
- 18: Ventrales Fenster
- 20: Dorsales Fenster
- 22: Bohrung
- 24: Bistabile Matrix
- 26: Distale Spange
- 28: Ventilöffnung

## Patentansprüche

1. Prothesenschaft (1) für eine Prothese, der
Anschlussmittel für eine distale Protheseneinrichtung und
einen Außenschaft (2) aufweist, der
überwiegend aus einem starren Material gebildet ist und
eine proximale Öffnung (4) zum Aufnehmen eines Amputationsstumpfes umfasst, die von einem Außenschaftrand (6) umgeben ist, wobei
der Außenschaftrand (6) in einem ventralen Bereich aus einem flexiblen und unelastischen Material (16) gebildet ist, das so ausgebildet ist, dass es unter Einwirkung der herkömmlicherweise beim Tragen des Prothesenschaftes auftretenden Belastungen zu flexiblen Verformungen, nicht jedoch zu einer Aufweitung der proximalen Öffnung (4) kommt, wobei das flexible und unelastische Material (16) an eine erwartete Körperform eines Trägers des Prothesenschaftes (1) anpassbar ist, wobei diese Form nach Ausformen des flexiblen und unelastischen Materials (16) eine Ruheposition des flexiblen und unelastischen Materials (16) bildet, in die es zurückkehrt, sofern es nicht anderen Kräften unterworfen ist.

2. Prothesenschaft (1) nach Anspruch 1, **dadurch gekennzeichnet, dass** das flexible und unelastische Material ein faserverstärkter Kunststoff ist, der insbesondere Dyneema®-Fasern umfasst.

3. Prothesenschaft (1) nach Anspruch 1 oder 2, **dadurch gekennzeichnet, dass** das starre Material ein faserverstärkter Kunststoff ist.

4. Prothesenschaft (1) nach Anspruch 3, **dadurch gekennzeichnet, dass** das starre Material und das flexible und unelastische Material (16) den gleichen Kunststoff umfasst.

5. Prothesenschaft (1) nach einem der vorstehenden Ansprüche, **dadurch gekennzeichnet, dass** der Außenschaftrand (6) in einem dorsalen Bereich aus dem flexiblen und unelastischen Material (16) gebildet ist.

6. Prothesenschaft (1) nach Anspruch 5, **dadurch gekennzeichnet, dass** das flexible und unelastische Material (16) im dorsalen Bereich des Außenschaftrandes (6) das gleiche Material ist, wie das flexible und unelastische Material (16) im ventralen Bereich des Außenschaftrandes (6).

7. Prothesenschaft (1) nach einem der vorstehenden Ansprüche, **dadurch gekennzeichnet, dass** der Außenschaft (2) an zumindest einer Stelle durchbrochen ist.

8. Prothesenschaft (1) nach einem der vorstehenden Ansprüche, **dadurch gekennzeichnet, dass** in dem Außenschaft (2) ein Innenschaft (8) aus einem flexiblen Material angeordnet ist.

9. Prothesenschaft (1) nach Anspruch 8, **dadurch gekennzeichnet, dass** der Innenschaft (8) mit dem Außenschaft (2) hubfrei verbunden ist.

10. Prothesenschaft (1) nach Anspruch 9, **dadurch gekennzeichnet, dass** der Innenschaft (8) mit dem Außenschaft (2) durch wenigstens eine insbesondere distale Schraubverbindung verbunden ist.

## Claims

1. A prosthesis socket (1) for a prosthesis comprising an attachment member adapted to be connected to a distal prosthetic device and an outer socket (2) which is formed mainly from a rigid material and comprises a proximal opening (4) for receiving an amputation stump, said proximal opening being surrounded by an outer-socket edge (6), the outer-socket edge being formed from a flexible and non-elastic material (16) in a ventral region, the flexible and non-elastic material being adapted such that flexible deformations can take place under the effect of the loads that customarily occur when wearing the prosthesis socket, but the proximal opening (4) does not widen, the flexible and non-elastic material being adapted to a body shape of a wearer of the prosthesis socket (1), wherein the shape after forming the flexible and non-elastic material (16) forms the rest position of the flexible and non-elastic material (16) which it returns to if not subjected to other forces.

2. The prosthesis socket (1) as claimed in claim 1, **characterized in that** the flexible and non-elastic material is a fiber-reinforced plastic comprising Dyneema® fibers.

3. The prosthesis socket (1) as claimed in claim 1 or 2, **characterized in that** the rigid material is a fiber-reinforced plastic.

4. The prosthesis socket (1) as claimed in claim 3, **characterized in that** the rigid material and the flexible and non-elastic material (16) comprise the same plastic.

5. The prosthesis socket (1) according to one of the preceding claims, **characterized in that** the outer-socket edge (6) is formed from the flexible and non-elastic material (16) in a dorsal region.

6. The prosthesis socket (1) as claimed in claim 5, **characterized in that** the flexible and non-elastic material (16) in the dorsal region of the outer-socket edge (6) is the same material as the flexible and non-elastic material (16) in the ventral region of the outer-socket edge (6).

7. The prosthesis socket (1) according to one of the preceding claims, **characterized in that** the outer socket (2) is interrupted at at least one place.

8. The prosthesis socket (1) according to one of the preceding claims, **characterized in that** an inner socket (8) comprising a flexible material is arranged in the outer socket (2).

9. The prosthesis socket (1) as claimed in claim 8, **characterized in that** the inner socket (8) is connected to the outer socket (2) in a manner free of pistoning.

10. The prosthesis socket (1) as claimed in claim 9, **characterized in that** the inner socket (8) is connected to the outer socket (2) by at least one screw connection comprising in particular a distal screw connection.

## Revendications

1. Emboîture de prothèse (1) pour une prothèse, qui comprend des moyens de raccordement pour un agencement de prothèse distale et une emboîture extérieure (2), laquelle est en majeure partie réalisée en un matériau rigide et présente une ouverture proximale (4) pour recevoir un moignon d'amputation, laquelle est entourée par une bordure d'emboîture extérieure (6), dans laquelle la bordure d'emboîture extérieure (6) est formée dans une région ventrale d'un matériau flexible et non élastique (16), lequel est réalisé de telle façon que sous l'action des charges qui se produisent habituellement lors du port de l'emboîture de prothèse il se produit des déformations flexibles mais cependant pas un élargissement de l'ouverture proximale (4), dans laquelle le matériau flexible et non élastique (16) est susceptible d'être ajusté à la forme corporelle attendue d'un porteur de l'emboîture de prothèse (1), et cette forme, après conformation du matériau flexible est non élastique (16) forme une position de repos du matériau flexible et non élastique (16) à laquelle il retourne dès qu'il n'est plus soumis à d'autres forces.

2. Emboîture de prothèse (1) selon la revendication 1, **caractérisée en ce que** le matériau flexible et non élastique est une matière plastique renforcée par des fibres, qui inclut en particulier des fibres connues sous la marque Dyneema®.

3. Emboîture de prothèse (1) selon la revendication 1 ou 2, **caractérisée en ce que** le matériau rigide est une matière plastique renforcée par des fibres.

4. Emboîture de prothèse (1) selon la revendication 3, **caractérisée en ce que** le matériau rigide et le matériau flexible et non élastique (16) incluent la même matière plastique.

5. Emboîture de prothèse (1) selon l'une des revendications précédentes, **caractérisée en ce que** la bordure d'emboîture extérieure (6) est formée dans une région dorsale à partir du matériau flexible et non élastique (16).

6. Emboîture de prothèse (1) selon la revendication 5, **caractérisée en ce que** le matériau flexible et non élastique (16) est, dans la région dorsale de la bordure d'emboîture extérieur (6), le même matériau que le matériau flexible et non élastique (16) dans la région ventrale de la bordure d'emboîture extérieure (6).

7. Emboîture de prothèse (1) selon l'une des revendications précédentes, **caractérisée en ce que** l'emboîture extérieure (2) est ajourée à au moins un emplacement.

8. Emboîture de prothèse (1) selon l'une des revendications précédentes, **caractérisée en ce qu'**une emboîture intérieure (8) en un matériau flexible est agencée dans l'emboîture extérieur (2).

9. Emboîture de prothèse (1) selon la revendication 8, **caractérisée en ce que** l'emboîture intérieure (8) est reliée à l'emboîture extérieur (2) sans possibilité de se soulever.

10. Emboîture de prothèse (1) selon la revendication 9, **caractérisée en ce que** l'emboîture intérieure (8) est reliée à l'emboîture extérieure (2) par au moins une liaison vissée, en particulier distale.
